(19) Europäisches Patentamt
European Patent Office
Office européen des brevets

(11) Publication number: **0 329 337 A2**

(12) **EUROPEAN PATENT APPLICATION**

(21) Application number: **89301270.8**

(22) Date of filing: **09.02.89**

(51) Int. Cl.⁴: **C07C 53/02 , C07C 51/02 , C07C 85/24**

(30) Priority: **17.02.88 GB 8803649**
**01.03.88 GB 8804786**
**05.03.88 GB 8805309**

(43) Date of publication of application:
**23.08.89 Bulletin 89/34**

(84) Designated Contracting States:
**BE DE FR GB IT NL**

(71) Applicant: **BP Chemicals Limited**
**Belgrave House 76 Buckingham Palace Road**
**London, SW1W 0SU(GB)**

Applicant: **The British Petroleum Company p.l.c.**
**Britannic House Moor Lane**
**London EC2Y 9BU(GB)**

(72) Inventor: **Green, Michael James**
**The British Petroleum Company p.l.c.**
**Chertsey Road**
**Sunbury-on-Thames Middlesex TW16 7LN(GB)**
Inventor: **Lucy, Andrew Richard**
**The British Petroleum Company p.l.c.**
**Chertsey Road**
**Sunbury-on-Thames Middlesex TW16 7LN(GB)**
Inventor: **Kitson, Melanie**
**BP Chemicals Limited Salt End**
**Hedon Hull HU12 8DS(GB)**
Inventor: **Smith, Stephen James**
**BP Chemicals Limited Salt End**
**Hedon Hull HU12 8DS(GB)**

(74) Representative: **Richardson, Derek et al**
**BP INTERNATIONAL LIMITED Patents & Agreements Division Chertsey Road**
**Sunbury-on-Thames Middlesex TW16 7LN(GB)**

(54) **The production of formic acid from a nitrogenous base, carbon dioxide and hydrogen.**

(57) A process for the production of formic acid comprises (a) in a first stage reacting together a nitrogenous base, carbon dioxide and hydrogen in the presence of a catalyst to produce a formate salt of the nitrogenous base, (b) in a second stage removing from the formate salt of the nitrogenous base and any low-boilers co-produced therewith the catalyst and recycling this to the first stage, and in a subsequent stage or stages converting the formate salt of the nitrogenous base to formic acid. The process is characterised by the fact that after production of the formate salt of the nitrogenous base in stage (a) and either before or during the second stage (b) the catalyst is treated with a formate salt decomposition inhibitor which is either (I) a carboxylic acid or a salt thereof, (II) carbon monoxide or (III) an oxidant.

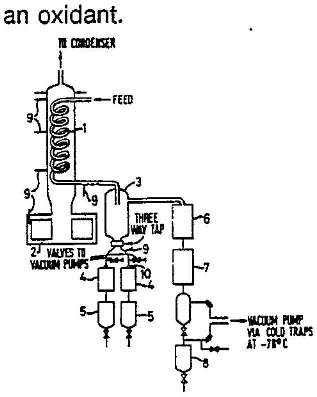


EP 0 329 337 A2

# THE PRODUCTION OF FORMIC ACID FROM A NITROGENOUS BASE, CARBON DIOXIDE AND HYDROGEN

The present invention relates to improvements in an integrated process for the production of formic acid from a nitrogenous base, carbon dioxide and hydrogen.

European patent applications publication Nos. 95321 and 126524 respectively describe a method for the production of a trialkylammonium formate from a tertiary amine, carbon dioxide and hydrogen and a method for converting the trialkylammonium formate into another formate salt which is thermally decomposable to formic acid.

European patent application publication No. 0181078 discloses an integrated process for the production of formic acid from carbon dioxide and hydrogen characterised in that

(a) in a first stage a nitrogeneous base, carbon dioxide and hydrogen are reacted together in the presence of a catalyst to produce a formate salt of the nitrogeneous base,

(b) in a second stage the catalyst is removed from the formate salt of the nitrogenous base and any low boilers and recycled to the first stage,

(c) in a third stage the formate salt of the nitrogenous base is recovered from the low boilers,

(d) in a fourth stage the formate salt of the nitrogenous base is reacted with a base having a high boiling point to produce the nitrogenous base and the formate salt of the base having a high boiling point, and

(e) in a fifth stage the formate salt of the base having a high boiling point is decomposed to the higher boiling base and formic acid.

In the first stage (a) of the process of EP-A-0181078 a high-boiling solvent is generally employed. In the second stage (b) of the process the catalyst and the high-boiling solvent is removed from the product of the first stage comprising unreacted materials, the formate salt of the nitrogenous base and catalyst in the high-boiling solvent. In a preferred arrangement the second stage comprises (a′) an evaporator wherein (i) the catalyst and high-boiling solvent are separated and recycled to the first stage reactor, and (ii) gaseous components are separated and recycled, followed by (c) a unit for the separation of unreacted nitrogenous base and water from the formate salt of the nitrogenous base. A problem can occur in the operation of the evaporator in that under the conditions of elevated temperature and low pressure prevailing therein the presence of catalyst together with the formate salt of the nitrogenous base can cause the reverse reaction to occur, i.e. the decomposition of the formate salt of the nitrogenous base to carbon dioxide and hydrogen, thereby decreasing the yield of the desirable formate salt. This problem is not restricted to operation using an evaporator for separation of the catalyst and high-boiling solvent, but may be encountered in any separation in which the catalyst remains in contact with the formate salt under conditions facilitating formate salt decomposition.

It is observed in a paper in the A.C.S. Symposium Series, Vol. 152, (1981) entitled "Mechanistic Aspects of the Homogeneous Water Gas Shift Reaction" by W.A.R. Slegeir, R.S. Sapienza and B. Easterling that the presence of high pressure carbon monoxide apparently inhibits the rate of formate decomposition in the presence of ruthenium carbonyl.

We have now surprisingly found that the problem of decomposition of the formate salt of the nitrogenous base can be substantially reduced by treating the catalyst after production of the formate salt of the nitrogenous base in the first stage (a) and either before or during removal of the catalyst from the formate salt of the nitrogenous base in the second stage (b) with a formate salt decomposition inhibitor which is either (I) a carboxylic acid or a salt thereof, (II) carbon monoxide or (III) an oxidant.

Accordingly the present invention provides a process for the production of formic acid by (a) in a first stage reacting together a nitrogenous base, carbon dioxide and hydrogen in the presence of a catalyst to produce a formate salt of the nitrogenous base, (b) in a second stage removing from the formate salt of the nitrogenous base and any low boilers co-produced therewith the catalyst and recycling this to the first stage, and in a subsequent stage or stages converting the formate salt of the nitrogenous base to formic acid

characterised in that

after production of the formate salt of the nitrogenous base in stage (a) and either before or during the second stage (b) the catalyst is treated with a formate salt decomposition inhibitor which is either (I) a carboxylic acid or a salt thereof, (II) carbon monoxide or (III) an oxidant.

The nitrogenous base may suitably be one containing a tertiary nitrogen atom having either the formula:-

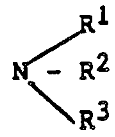

(I)

or the formula:-

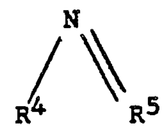

(II)

wherein in the formulae, $R^1$, $R^2$ and $R^3$, which may be the same or different, are hydrocarbyl groups or substituted hydrocarbyl groups or any two or all of $R^1$, $R^2$ and $R^3$ may form part of a ring, $R^4$ is a hydrocarbyl group or substituted hydrocarbyl group and $R^5$ is a divalent organic group or $R^4$ and $R^5$ may form part of a ring.

Suitably the hydrocarbyl group is an aliphatic, cycloaliphatic, aryl or alkaryl group. Substituted hydrocarbyl groups may contain for example nitrogen or oxygen. Preferably the nitrogenous base is a trialkylamine, even more preferably a lower alkylamine, for example a $C_1$ to $C_{10}$ trialkylamine. Examples of suitable trialkylamines include trimethylamine, triethylamine, tripropylamine and tributylamine. Examples of other suitable nitrogenous bases include amidines, for example 1,8-diazobicyclo[5.4.0]undec-7-ene (DBU) and 1,4-diazabicyclo[2.2.2]octane (DABCO), pyridine and picolines. The process of the invention will be found particularly applicable to, for example, triethylammonium formate as the formate salt of a nitrogenous base.

The formate salt of the nitrogenous base may suitably be prepared by the process described in our European application publication No. 0 095 321 in which hydrogen and carbon dioxide are reacted with a nitrogenous base, in the presence of a solvent and as catalyst a soluble compound of a transition metal of Group VIII of the Periodic Table according to Mendeleef and separating the formate salt of the base from the reaction mixture.

As catalyst there is used a compound of a Group VIII transition metal, which is preferably either iron, nickel, ruthenium, rhodium, palladium, iridium or platinum. More preferably the metal is ruthenium. Mixtures of compounds of different transition metals may also be used if so desired. The metal or metals may be added in any convenient form which is soluble in the reaction mixture. Thus the metal or metals may be added in the form of a simple salt, for example a halide, or in the form of a complex, for example a hydride complex. Examples of suitable ruthenium compounds which may be employed as catalyst are $RuCl_2(PPh_3)_3$, $RuH_2((PPh_3)_4$, $RuHCl(PPh_3)_4$, $RuCl_3.3H_2O$, $[Ru(CO)_2Cl_2]_n$, $[Ru(CO)_2I_2]_n$, $[(p\text{-cymene})RuCl_2]_2$, $[Ru(CO)_3Cl_2]_2$ $[(hexamethylbenzene)RuCl_2]_2$ and $[(hexamethylbenzene)Ru_2(OH)_3]Cl$ and $Ru_3(CO)_{12}$. Suitably the catalyst concentration may be in the range 50 to 5,000, prefeably from 250 to 1,000 parts per million by weight.

Generally, the rate of formate salt decomposition increases with increasing temperature.

The inhibitor may be a carboxylic acid or a salt thereof (i). The carboxylic acid may suitably be either a mono-, di- or poly-carboxylic acid, which may be either saturated or unsaturated and either aliphatic or aromatic, preferably aliphatic. Preferred inhibitors include dicarboxylic acids and their alkali metal salts. Examples of inhibitors suitable for use in the method of the invention include potassium acetate, disodium oxalate, disodium succinate, disodium malonate, oxalic acid and citric acid. A preferred inhibitor is oxalic acid. Mixtures of carboxylic acids and alkali metal salts of carboxylic acids may also be employed.

Suitably the inhibitor (I) may be added in a molar amount as compared with moles of metal in the catalyst of from 0.1 to 100:1, preferably from 0.5 to 10:1.

Using a carboxylic acid or an alkali metal salt thereof as the formate salt decomposition inhibitor, the catalyst may be deactivated both for the formation and decomposition of formate salts and it may therefore not always be possible to recycle it to the first stage directly without an intermediate reactivation step. Methods for recovering Group VIII transition metals from spent catalysts and reconverting them to active catalysts are however well-known to persons skilled in the art.

Carbon monoxide (II) may also be used as the inhibitor. Commerically available carbon monoxide may be employed with or without further purification. Impurities which may be present in the carbon monoxide include carbon dioxide, hydrogen, nitrogen and gaseous paraffinic hydrocarbons, for example methane.

Another class of inhibitor (III) is an oxidant Desirably the oxidant should be (i) inexpensive, (ii) thermally stable, (iii) involatile and (iv) reducible to harmless products, for example water, which are readily separable.

Suitable oxidants having at least one of the properties (i) to (iv) include hydrogen peroxide, alkyl or aryl peroxides, dialkyl peroxides, peracids, amine oxides, oxygen, copper (I) chloride/oxygen, sodium hypochlorite, chlorates, periodates and persulphates. Of the aforesaid oxidants hydrogen peroxide is a preferred oxidant for the reasons of effectiveness, low cost and separation of the co-product (water). On the other hand, if higher costs can be tolerated, amine oxides are preferred because of their involatility, thermal stability (long-lasting activity) and low reactivity towards other reaction components. Mixtures of oxidants providing a combination of the desirable properties (i) to (iv) may be used. A preferred amount of the oxidant is in the range from 1 to 20 moles of oxidant for every mole of Group VIII transition metal, for example ruthenium, present.

The catalyst species resulting from treatment with either carbon monoxide or an oxidant is not only substantially inactive for formate salt decomposition, it is also substantially inactive for formate salt formation. However, the inactivation of the catalyst is only temporary, the inhibited catalyst reverts in situ to an active form. This is an advantage of using either carbon monoxide or an oxidant as the inhibitor,since it allows re-use of the catalyst after separation from the reaction mixture without any intervening step, though it may be desirable to perhaps heat the temporarily inactivated catalyst to accelerate its reactivation. The lifetime of the temporarily inactivated catalyst species depends on the temperature, the amount and type of inhibitors (II) or (III) and the nature and composition of the reactants, for example the ratio of nitrogenous base to formic acid. From the aforesaid inhibitors (II) or (III) it is possible to select one which inhibits formate salt decomposition for a period sufficient to allow removal of the formate salt from any given reaction mixture and to thereafter allow the catalyst to be re-used in the production of the formate salt on recyle.

It is preferred to convert the formate sale of the nitrogenous base to formic acid by the steps comprising:-

(c) in a third stage recovering the formate salt of the nitrogenous base from any low boilers,

(d) in a fourth stage reacting the formate salt of the nitrogenous base recovered in stage (c) with a base having a high boiling point to produce the nitrogenous base and a formate salt of the base having a high boiling point, and

(e) in a fifth stage decomposing the formate salt of the base having a high boiling point to the higher boiling base and formic acid.

However, other methods for converting the formate salt of a nitrogenous base resulting from stages (a) and (b) to formic acid may be employed. For example, the formate salt of the nitrogenous base may be recovered from any low boilers and thereafter thermally decomposed under subatmospheric pressure conditions.

As regards the reactants, the reaction conditions and the equipment useful in the operation of the improved integrated process of the invention, the reader is referred to the disclosure of the aforesaid EP-A-0181078, which is incorporated herein by reference.

The process of the present invention will now be further illustrated by reference to the following examples.

Two types of experiment were performed, the first type being directed to the effect of inhibitors on the rate of decomposition of formic acid (as triethylammonium formate) in the presence of a ruthenium catalyst and the second type being flash evaporator experiments.

## (A) EFFECT OF INHIBITORS

### KINETIC EXPERIMENTS

### Comparison Test 1 and Examples 1 - 10

The feed mixtures for the kinetic experiments were prepared in a 300 ml capacity stainless steel autoclave fitted with a rotary stirrer. Table 1 details the liquid and solid charges to the autoclave. After charging, the autoclave was closed and carbon dioxide gas introduced with stirring until a pressure of 27 barg was maintained. The autoclave was then heated to 80°C and hydrogen admitted to a pressure of 95 barg. The fall in pressure against time was monitored and the heater was switched off after the gas uptake had ceased or after eight hours, whichever was the shorter. After the reactor had cooled to ambient

temperature it was depressurised and drained. This procedure is known to generate triethyl ammonium formate. 8 ml of formic acid was added to the product which resulted in the overall formic acid:triethylamine ratio being approximately 2:1. This was found necessary because there are two different decomposition rates; depending on whether the formic acid or triethylamine is in excess.

In a typical kinetic experiment the product from the autoclave, with the extra 8 ml of formic acid, was placed in a 250 ml round bottom flask fitted with a condenser and rotary stirrer, situated in an oil bath thermostated to 95 °C. Approximately 2 ml samples were taken every 10 minutes, including one of the starting mixture, for 90 minutes. Immediately after removal the sample was quickly cooled in a solid carbon dioxide/acetone bath to stop the decomposition reaction. It was stored at -30 °C until the decomposition run was complete. All eleven samples were then analysed for formic acid and/or triethylammonium formate composition by hydrolysing with Amberlyst A15 ion exchange resin followed by base titration. Previous GLC analysis has shown formic acid to be the only acid present.

From these figures the zero order rate of decomposition (found in acid rich conditions) and the first order rate constant (found in base rich conditions) were calculated. These are found in Table 2. All the additives decrease the rate of formate decomposition. The best inhibitor tested is oxalic acid which reduced the decomposition rate under formic acid rich conditions to 9% and base rich conditions to 4% of baseline.

## OTHER EXPERIMENTS

### Comparison Test 2

A 115 $cm^3$ Fisher-Porter glass pressure vessel containing a magnetic stirring bar was charged with tetraethylene-glycol (7.12 g), water (0.08 g), triethylamine (1.02 g) and formic acid (0.73 g). The mixture was allowed to cool and $[Ru(CO)_3Cl_2]_2$ (12.5 mg) was then added. The vessel was attached to a pressure line, flushed with nitrogen and then partly immersed in an oil bath maintained at 130 °C. The mixture was stirred and the pressure monitored until gas evolution ceased.

### Examples 11 - 17

These were carried out as in Comparison Test 2 with the addition of a measured amount of oxidant.

### Comparison Test 3

This was carried out as in Comparison Test 2 except that 2.05 g of triethylamine was employed.

### Example 18

This was carried out as in Comparison Test 3 with the addition of hydrogen peroxide.

### Comparison Test 4

Comparison Test 3 was repeated except that the oil bath was maintained at 100 °C.

### Example 19

Example 18 was repeated except that the oil bath was maintained at 100 °C.

The results of Examples 11 to 19 and Comparison Tests 2 to 4 are given in the accompanying Table 3.

Comparison Tests 2 to 4 are not examples according to the invention because no oxidant was employed. They are included only for the purpose of comparison.

TABLE 1

| Example | Additive | Charge/g | | | | |
|---|---|---|---|---|---|---|
| | | TEG+ | $NEt_3$ | $H_2O$ | $[Ru(CO)_2Cl_2]_n$ | Additive |
| CT 1 | | 130.9 | 36.3 | 5.4 | 0.1976 | |
| 1 | Potassium Acetate | 128.8 | 37.9 | 5.3 | 0.2072 | 0.0859 |
| 2 | Potassium Acetate | 128.0 | 38.6 | 5.2 | 0.2082 | 0.1826 |
| 3 | Disodium Oxalate | 131.6 | 36.8 | 5.5 | 0.2047 | 0.0611 |
| 4 | Disodium Oxalate | 128.1 | 39.6 | 5.4 | 0.2141 | 0.1253 |
| 5 | Disodium Oxalate | 128.8 | 36.3 | 5.5 | 0.1973 | 0.2503 |
| 6 | Disodium Succinate | 129.6 | 36.6 | 5.2 | 0.1900 | 0.1527 |
| 7 | Disodium Malonate $2H_2O$ | 130.1 | 36.5 | 5.3 | 0.1814 | 0.15* |
| 8 | Oxalic Acid $2H_2O$ | 130.9 | 37.1 | 5.3 | 0.2010 | 0.0589 |
| 9 | Oxalic Acid $2H_2O$ | 128.3 | 36.4 | 5.4 | 0.2039 | 0.1225 |
| 10 | Citric Acid $H_2O$ | 130.4 | 36.8 | 5.3 | 0.1985 | 0.1850 |

* Charge solution saturated
\+ TEG - tetraethylene glycol
CT - Comparison Test

TABLE 2

| Example | Additive | | Zero Order Rate | | First Order Rate Constant | |
|---|---|---|---|---|---|---|
| | Type | Equivalence+ | $molkg^{-1}h^{-}$ | % Baseline | $min^{-1}$ | % Baseline |
| CT 1 | | | 3.59* | 100 | 0.05* | 100 |
| 1 | $KOCOCH_3$ | 1.0 | 1.16 | 32 | 0.018 | 36 |
| 2 | $KOCOCH_3$ | 2.0 | 2.24 | 62 | 0.025 | 50 |
| 3 | $Na_2(OCO)_2$ | 0.5 | 3.76 | 104 | 0.037 | 74 |
| 4 | $Na_2(OCO)_2$ | 1.0 | 1.40 | 39 | 0.012 | 24 |
| 5 | $Na_2(OCO)_2$ | 2.0 | 1.20 | 33 | 0.010 | 20 |
| 6 | $Na_2(OCOCH_2)_2$ | 1.0 | 2.11 | 59 | 0.026 | 52 |
| 7 | $Na_2(OCOCH_2)_2\ 2H_2O$ | 1.0 | 1.68 | 47 | 0.026 | 52 |
| 8 | $(HOCO)_2.2H_2O$ | 0.5 | 2.48 | 69 | 0.024 | 48 |
| 9 | $(HOCO)_2.2H_2O$ | 1.0 | 0.31 | 9 | 0.002 | 4 |
| 10 | $C(OH)(CO_2H)\ (CH_2CO_2H)_2.H_2O$ | 1.0 | 3.49 | 97 | 0.006 | 12 |

* The average of four repeats of Example 1
\+ Approximate molar equivalence to ruthenium
CT - Comparison Test

Table 3

| Example | Oxidant (Equivalents per Ru) | Pressure (bar)[a] | | |
|---|---|---|---|---|
| | | 7 minutes | 11 minutes | 15 minutes |
| CT 2 | - | 2.50 | 6.30 | 6.60 |
| 11 | $Bu^tOOH$ (1) | 0.55 | 4.70 | 6.55 |
| 12 | $Bu^tOOH$ (2) | 0.25 | 2.70 | 6.40 |
| 13 | $Bu^tOOH$ (10) | 0.15 | 0.20 | 4.95 |
| 14 | $H_2O_2$ (10) | 0.05 | 1.30 | 5.95 |
| 15 | $H_2O_2$ (5) + $Me_3NO$ (5) | 0.05 | 0.35 | 1.30 |
| 16 | N-methylmorpholine - N-oxide (10) | 0.20 | 0.40 | 0.75 |
| 17 | $Me_3NO$ | 0.35 | 0.55 | 1.00 |
| CT 3 | - | 3.80 | 6.10 | 6.20 |
| 18 | $H_2O_2$ (10) | 0.10 | 1.40 | 6.35 |
| CT 4 | - | 1.20 | 3.00 | 4.65 |
| 19 | $H_2O_2$ (10) | 0.15 | 0.30 | 0.65 |

[a] Corrected for pressure observed in absence of $[Ru(CO)_3Cl_2]_2$

(B) VACUUM EVAPORATOR EXPERIMENTS

For these experiments the vacuum evaporator illustrated in the accompanying Figure was employed.

With reference to the Figure, 1 is a glass coil (dimensions -12 turns of pitch 25mm, 25mm radius, 8mm internal diameter), 2 is a glass reboiler wrapped with a heating element, 3 is a vapour/liquid knockout pot, 4 are cold water condensers, 5 are base take-off vessels, 6 is a cold water condenser, 7 is a refrigerated condenser, 8 is a heads take-off vessel, 9 are thermocouples and 10 is a pressure indicator.

After the runs were complete the three streams: base take-off, heads take-off and cold trap drainings plus the feed were analysed for overall acid/base excess by titration, for total formic acid/formate content by passing over a column of Amberlyst 15 ion-exchange resin to liberate formic acid, then titrating against base and for water content by Karl Fischer analysis.

(i) Carbon monoxide as the inhibitor

In Comparison Test 5 and Examples 24 and 25 the rate of reaction refers to the rate of production of the formate salt (moles/hour) divided by the weight of reaction solution (kg). The conversion to formate salt was calculated according to the following equation:
conversion = 100 x moles of formate produced/moles of nitrogenous base added.

Comparison Test 5

The feed was prepared by mixing together 1700.6 g tetraethylene glycol, 495.4 g triethylamine, 142.5 g water, 167.2 g formic acid and 2.536 g $[Ru(C0)_2Cl_2]_n$. Before starting to add the feed the heaters and coolers on the evaporator were allowed to gain their working temperatures:reboiler = 100 °C, vapour-liquid knockout pot 100 °C, fridge condensers = -25 °C and the evaporator evacuated. A 0.5 h pre-run was necessary to coat the internal surfaces and let equilibrium conditions be attained. The run was started by switching over to a separate set of collection vessels. After a known mass of feed had been pumped through the system the original set of collection vessels were switched back. Details of the process streams are given in Table 4, and the results in Table 10.

Comparison Test 5 is not an example according to the invention because carbon monoxide was not employed. It is included only for the purpose of comparison.

Example 20

Into an autoclave of 1 litre capacity made of stainless steel and fitted with a rotary stirrer were charged 85.9 g tetraethylene glycol, 401.0 g triethylamine, 57.8 g water, 122.0 g formic acid and 2.1905 g $[Ru(CO)_2Cl_2]_n$. The autoclave was closed and carbon monoxide was introduced until a pressure of 50 barg was obtained. After 15 h the stirrer was switched on and the autoclave heated to 70°C for 1.5 h with stirring. The autoclave was then rapidly cooled to 30°C and depressurised. The reaction mixture was added to 1350 g tetraethylene glycol. This solution was run on the evaporator within 4 h of being drained from the autoclave. The procedure used was that described in Comparison Test 5 except that the pre-run duration was 0.90 h and the run duration was 0.77 h. The stream compositions for this run are given in Table 5 and the results in Table 10.

Example 21

The feed mixture was prepared in a similar manner to that described in Example 20 with the total quantities used being 1431.7 g tetraethylene glycol, 400.1 g triethylamine, 58.7 g water, 120.2 g formic acid and 2.2022 g $[Ru(C0)_2Cl_2]_n$. In this example, performed as described in Comparison Test 5, the pre-run duration was 1.48 h and the run time 1.57 h. Table 6 contains the stream compositions. From Table 10 it can be seen that, compared to Comparison Test 5, there has been a six-fold reduction in the amount of formate decomposition while the formate recovery is similar.

Example 22

The feed mixture was prepared in a similar manner to that described in Example 20 with the total quantities used being 1433.8 g tetraethylene glycol, 406.4 g triethylamine, 60.2 g water, 128.0 g formic acid and 2.1929 g $[Ru(C0)_2Cl_2]_n$. This run, performed as described in Comparison Test 5, used a slow feed rate and had pre-run and run durations of 3.25 and 2.90 h. The stream compositions are detailed in Table 7.

The results in Table 10 show a substantial increase in formate recovery with only a small increase in formate decomposition.

Comparison Test 6

The feed was prepared by mixing together 1435.1 g tetraethylene glycol, 358.0 g triethylamine, 69.1 g water, 121.6 g formic acid and 2.0293 g $[Ru(C0)_2Cl_2]_n$. This run was performed as described in Comparison Test 5 except that the reboiler temperature was 138°C, giving an average evaporator skin temperature of approximately 122°C. The pre-run and run durations were 1.97 and 2.57 h, respectively. The stream compositions are found in Table 8 with the results in Table 10.

Example 23

The feed was prepared in a similar manner to that described in Example 20 with the total quantities used being: 1419.0 g tetraethylene glycol, 396.5 g triethylamine, 67.2 g water, 130.7 g formic acid and 2.17 g $[Ru(C0)_2Cl_2]_n$. This run was performed as described in Comparispm Test 6. The stream composition are detailed in Table 9 and the pre-run and run duration were 2.07 and 2.57 h, respectively. The results in Table 10 show that compared to Comparison Test 6 there has been significantly more formate recovered, with less decomposition.

Comparison Test 7

The base take-off from Comparison Test 6 (138.15 g) was mixed with triethylamine (39.90 g) and water (5.30 g) and placed in a 300 ml stainless steel autoclave fitted with a magnedrive stirrer and thermocouple. This was purged and then saturated at 400 psig with carbon dioxide. After heating to 80°C the autoclave was charged to 1400 psig total with hydrogen. The fall in pressure with time was monitored. The autoclave

was maintained at 80°C until gas consumption ceased and then cooled and vented. The liquid product was analysed by passing it over an Amberlyst 15 ion exchange column to liberate formic acid followed by titration against base. Gas chromatographic analysis of similar products have shown that the only product is formic acid. The conversion was 63.7% and the productivity was 7.60 mol/kg/h.

Example 24

The base take off of Example 22 (130.51 g) was mixed with triethylamine (38.92 g) and water (5.32 g) and the procedure of Comparison Test 7 was followed. The conversion to formate was 58.4% and the productivity was 5.19 mol/kg/h. After taking into account the relative ruthenium contents of Comparison Test 7 and Example 24 this shows the recycled catalyst to have 70.3% of the activity of a non-carbon monoxide treated catalyst.

Example 25

A typical product mixture was heated to 80°C under vacuum on a rotary evaporator for 3 hours to remove the volatile materials. 128.8 g of this was then charged to the autoclave with triethylamine (37.9 g) and water (5.2 g) and treated as described in Comparison Test 7. The resulting solution contained 1.52 $mmolg^{-1}$ formate, which corresponds to a productivity of 6.93 mol $kg^{-1}h^{-1}$ with $NEt_3$ conversion of 69.0%. After taking into account the relative ruthenium contents of Comparison Test 7 and Example 25 this shows the recycled catalyst to have 79.5% of its original activity.

(ii) A carboxylic acid or a salt thereof as inhibitor

Comparison Test 8

The feed was prepared by mixing together tetraethylene glycol (1700.6 g), triethylamine (495.4 g), water (142.5 g) formic acid (167.2 g) and $[Ru(CO)_2Cl_2]_n$ (2.536 g). Before starting to add the feed the heaters and coolers on the evaporator were allowed to gain their working temperatures:reboiler = 100°C, vapour-liquid knockout pot = 100°C, fridge condensers = -25°C and the system evacuated. A 0.5 h pre-run was necessary to coat the internal surfaces and let equilibrium conditions be attained. The run was started by switching over to a separate set of collection vessels. After a known mass of feed had been pumped through the system the original set of collection vessels were switched back. Details of the process streams are given in Table 11, with the results in Table 14.

Example 26

The feed mixture was prepared by saturating at 80°C for 3 h a mixture of tetraethylene glycol (1871.0 g), triethylamine (573.7 g), water (79.9 g) and $[Ru(CO)_2Cl_2]_n$ (2.6763 g) with disodium oxalate. After cooling and filtering off the undissolved disodium oxalate formic acid (146.67 g) was mixed in. The procedure used was as that described in Comparison Test 8 except that the pre-run duration was 0.87 h and the run duration 1.00 h. Table 12 contains the data on the steam compositions. From Table 14 it can be seen that the amount of formate decomposition has been reduced in comparison to Comparison Test 8 by approximately a factor of two.

Example 27

The feed was prepared by boiling under reflux for 3 h a mixture of tetraethylene glycol (555.7 g), triethylamine (313.1 g), water (53.7 g), $[Ru(CO)_2Cl_2]_n$ (1.8032 g) and oxalic acid dihydrate (0.9624 g). When cool this solution was added to tetraethylene glycol (713.6 g) and to this was added formic acid (97.9 g). The run was performed as described in Comparison Test 8 except that the pre-run and run durations were 2.08 and 3.35 h, respectively, and the temperatures in the reboiler and vapour-liquid knockout pot were

increased. The stream compositions are detailed In Table 13 with the results in Table 14 showing significantly more formate recovered, compared to Comparison Test 8, with less decomposition.

Table 4

| Stream | Weight (g) | Composition/wt% | | |
|---|---|---|---|---|
| | | Water | TEA | FA |
| Feed | 1835.4 | 5.82 | 19.81 | 6.39 |
| Base T/O | 1238.9 | 0.16 | 2.03 | 1.24 |
| Heat T/O | 554.8 | 20.74 | 60.50 | 12.07 |

Table 5

| Stream | Weight (g) | Composition/wt% | | |
|---|---|---|---|---|
| | | Water | TEA | FA |
| Feed | 765.6 | 3.41 | 19.39 | 5.49 |
| Base T/O | 606.5 | 0.22 | 6.36 | 4.05 |
| Heat T/O | 151.4 | 12.72 | 74.15 | 10.43 |

Table 6

| Stream | Weight (g) | Composition/wt% | | |
|---|---|---|---|---|
| | | Water | TEA | FA |
| Feed | 735.7 | 3.33 | 19.32 | 5.55 |
| Base T/O | 559.3 | 0.15 | 3.93 | 2.75 |
| Heat T/O | 171.5 | 11.24 | 71.64 | 13.61 |

Table 7

| Stream | Weight (g) | Composition/wt% | | |
|---|---|---|---|---|
| | | Water | TEA | FA |
| Feed | 740.01 | 3.40 | 19.63 | 6.14 |
| Base T/O | 541.64 | 0.28 | 2.71 | 1.88 |
| Heat T/O | 192.75 | 13.21 | 65.60 | 16.92 |

Table 8

| Stream | Weight (g) | Composition/wt% | | |
|---|---|---|---|---|
| | | Water | TEA | FA |
| Feed | 710.00 | 4.03 | 17.72 | 5.85 |
| Base T/O | 505.09 | 0.19 | 0.11 | 0.28 |
| Heat T/O | 178.78 | 14.42 | 67.26 | 9.55 |

Table 9

| Stream | Weight (g) | Composition/wt% | | |
|---|---|---|---|---|
| | | Water | TEA | FA |
| Feed | 767.47 | 3.70 | 19.14 | 6.19 |
| Base T/O | 536.60 | 0.13 | 0.90 | 0.50 |
| Heat T/O | 212.89 | 12.59 | 66.76 | 13.50 |

Table 10

| Example | Feed Rate ($mlh^{-1}$) | Vacuum (m/bar) | Temp (°C) | [Ru] (ppm) | Efficiency of Formate Recovery (%) | Amount of Formate Decomposition (%) |
|---|---|---|---|---|---|---|
| CT 5 | 999 | 14 | 96 | 448 | 57.1 | 29.7 |
| 20 | 952 | 7 | 96 | 440 | 37.6 | 3.96 |
| 21 | 445 | 6 | 96 | 460 | 57.2 | 5.06 |
| 22 | 241 | 5 | 96 | 450 | 71.8 | 5.68 |
| CT 6 | 259 | 15 | 122 | 440 | 41.2 | 55.5 |
| 23 | 239 | 15 | 122 | 450 | 60.6 | 33.7 |
| CT = Comparison Test | | | | | | |

TABLE 11

| Stream | Weight g | Composition/wt% | | |
|---|---|---|---|---|
| | | Water | TEA | FA |
| Feed | 1835.4 | 5.82 | 19.81 | 6.39 |
| Base T/O | 1238.9 | 0.16 | 2.03 | 1.24 |
| Heat T/O | 554.8 | 20.74 | 60.50 | 12.07 |

TABLE 12

| Stream | Weight g | Composition/wt% | | |
|---|---|---|---|---|
| | | Water | TEA | FA |
| Feed | 1269.0 | 3.76 | 21.46 | 5.49 |
| Base T/O | 940.4 | 0.24 | 3.81 | 2.65 |
| Heat T/O | 311.8 | 11.63 | 73.57 | 11.26 |

TABLE 13

| Stream | Weight g | Composition/wt% | | |
|---|---|---|---|---|
| | | Water | TEA | FA |
| Feed | 751.7 | 3.49 | 17.69 | 4.77 |
| Base T/O | 545.3 | 0.27 | 1.61 | 1.05 |
| Heat T/O | 183.1 | 12.92 | 67.21 | 14.85 |

TABLE 14

| Example | Feed Rate $mlh^{-1}$ | Vacuum m bar | Temperature °C | [Ru] ppm | Efficiency of Formate Recovery % | Amount of Formate Decomposition % |
|---|---|---|---|---|---|---|
| CT 8 | 999 | 14 | 96 | 448 | 57.1 | 29.7 |
| 26 | 1250 | 9 | 96 | 444 | 50.5 | 13.7 |
| 27 | 212 | 15 | 122 | 460 | 76.8 | 8.2 |

## Claims

1. A process for the production of formic acid by (a) in a first stage reacting together a nitrogenous base, carbon dioxide and hydrogen in the presence of a catalyst to produce a formate salt of the nitrogenous base, (b) in a second stage removing from the formate salt of the nitrogenous base and any low-boilers co-produced therewith the catalyst and recycling this to the first stage, and in a subsequent stage or stages converting the formate salt of the nitrogenous base to formic acid
characterised in that
after production of the formate salt of the nitrogenous base in stage (a) and either before or during the second stage (b) the catalyst is treated with a formate salt decomposition inhibitor which is either (I) a carboxylic acid or a salt thereof, (II) carbon monoxide or (III) an oxidant.

2. A process according to claim 1 wherein the formate salt decomposition inhibitor is a carboxylic acid or a salt thereof.

3. A process according to claim 2 wherein the carboxylic acid is a dicarboxylic acid.

4. A process according to either claim 2 or claim 3 wherein the salt is an alkali metal salt.

5. A process according to any one of claims 2 to 4 wherein the inhibitor is oxalic acid.

6. A process according to claim 1 wherein the formate salt decomposition inhibitor is carbon monoxide.

7. A process according to claim 1 wherein the formate salt decomposition inhibitor is an oxidant.

8. A process according to claim 7 wherein the oxidant is either hydrogen peroxide, an alkyl or aryl peroxide, a dialkyl peroxide, a peracid, an amine oxide, oxygen, copper(I)chloride/oxygen, sodium hypochlorite, a chlorate, a periodate or a persulphate.

9. A process according to claim 7 wherein the oxidant is hydrogen peroxide.

10. A process according to claim 7 wherein the oxidant is an amine oxide.

11. A process according to any one of the preceding claims wherein the formate salt of the nitrogenous base is converted to formic acid by the steps comprising:-

(c) in a third stage recovering the formate salt of the nitrogenous base from any low boilers,

(d) in a fourth stage reacting the formate salt of the nitrogenous base recovered in stage (c) with a base having a high boiling point to produce the nitrogenous base and a formate salt of the base having a high boiling point, and

(e) in a fifth stage decomposing the formate salt of the base having a high boiling point to the higher boiling base and formic acid.

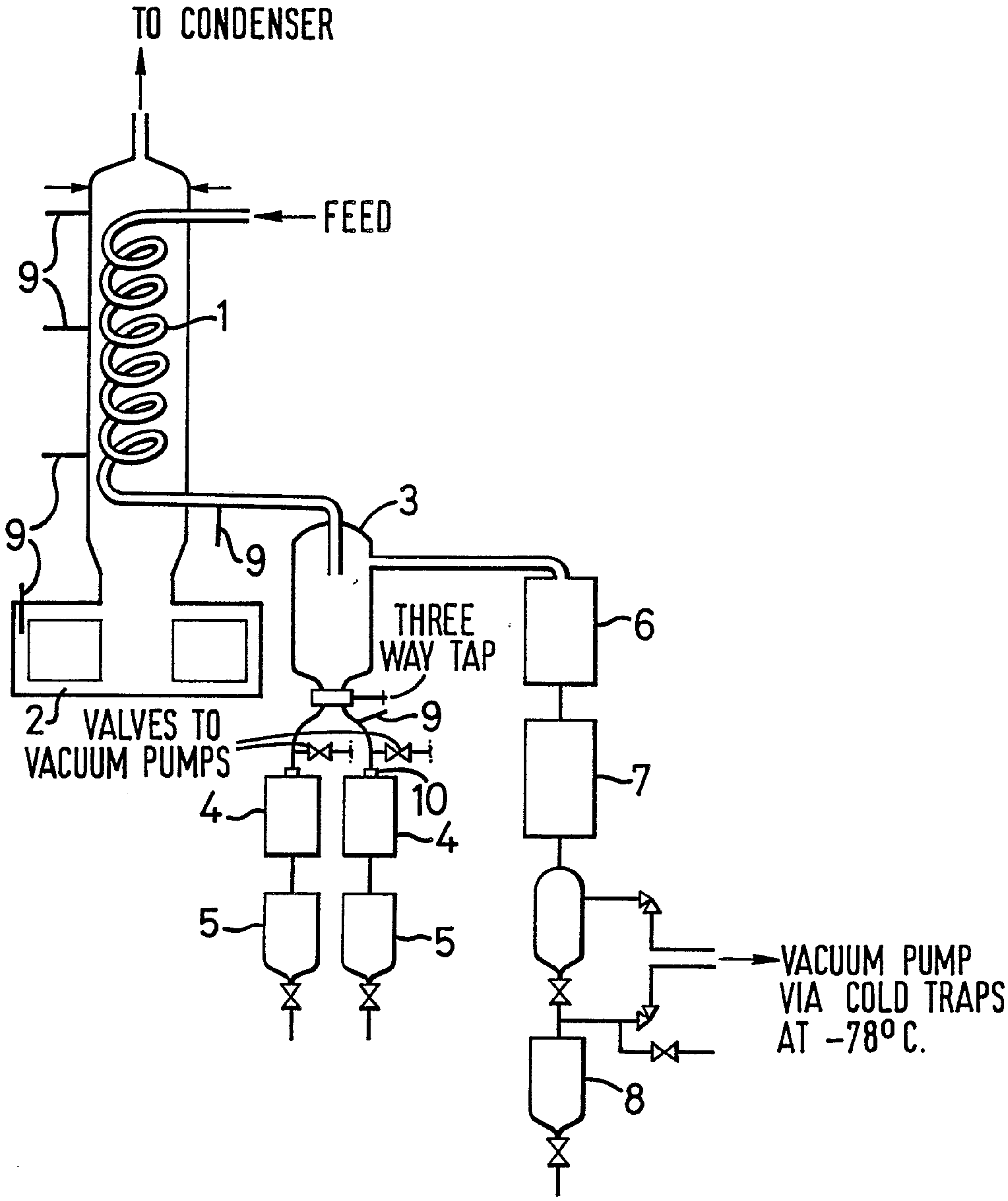
TO CONDENSER
FEED
9
1
9
3
9
THREE WAY TAP
6
2
VALVES TO VACUUM PUMPS
9
10
4
4
7
5
5
VACUUM PUMP VIA COLD TRAPS AT -78° C.
8